# EUROPEAN PATENT APPLICATION

(11) **EP 4 644 830 A1**
(43) Date of publication of application: **05.11.2025**
(21) Application number: 24905154.1
(22) Date of filing: 13.08.2024
(51) Int. Cl.: G01B 11/00, H01M 4/04, B05C 11/10, G01B 11/14

(54) **COATING MISALIGNMENT DETECTION METHOD AND APPARATUS, COMPUTER DEVICE, AND STORAGE MEDIUM**

(30) Priority: 08.02.2024 CN 202410175985
(71) Applicant: Contemporary Amperex Technology Co., Limited, Ningde, Fujian 352100 (CN)
(72) Inventor: HU, Liangjin, Ningde Fujian 352100 (CN); MA, Lin, Ningde Fujian 352100 (CN); ZHANG, Zili, Ningde Fujian 352100 (CN); WANG, Xi, Ningde Fujian 352100 (CN); SHI, Dewei, Ningde Fujian 352100 (CN)
(74) Representative: Gong, Jinping
(86) International application number: PCT/CN2024/111863
(87) International publication number: WO 2025/167038

(57) **Abstract**

The present application relates to a coating misalignment detection method, an apparatus, a computer device, and a storage medium. The method includes: obtaining a coating type of an electrode plate substrate and determining a number of reference edges according to the coating type; determining a corresponding target reference edge of each coating region edge of the electrode plate substrate in a case where the number of reference edges is two; determining first distance data from coating region edges on a first surface of the electrode plate substrate to a corresponding target reference edge and second distance data from coating region edges on a second surface of the electrode plate substrate to a corresponding target reference edge; and determining a coating misalignment amount of the electrode plate substrate in a coating process according to the first distance data and the second distance data. By adopting the method, the interference generated by blank regions on the surfaces of the electrode plate substrate in the coating misalignment detection process can be reduced, and the accuracy of the coating misalignment detection is effectively improved.

## Description

### CROSS-REFERENCE

The present application refers to Chinese Patent Application No. 2024101759855 entitled "COATING MISALIGNMENT DETECTION METHOD, APPARATUS, COMPUTER DEVICE, AND STORAGE MEDIUM" and filed on February 08, 2024, which is incorporated herein by reference in its entirety.

### TECHNICAL FIELD

The present application relates to the technical field of battery manufacturing, and in particular, to a coating misalignment detection method, an apparatus, a computer device, and a storage medium.

### BACKGROUND

The coating process of batteries is one of the most cutting-edge and most important processes in battery production. In the coating process, the stability, uniformity, size, and the like of the coating can influence the final performance of the battery.

When the continuous coating is carried out, there may be the shift of the electrode plate due to mechanical errors, guide roller errors, vibration, fluctuation in the tension of the electrode plate, and the like in the coating device, such that coatings on two surfaces of the electrode plate are misaligned in the coating process, the performance of the battery is seriously influenced, and thereby the production cost of the battery is increased.

In some cases, conventional coating misalignment detection methods usually only consider the misalignment between the coating regions and simply determine the difference value between the coating regions of the electrode plate, which easily causes the detection result to be interfered by, for example, the blank region, thereby reducing the accuracy of the detection result.

### SUMMARY

In view of the above, it is necessary to provide a coating misalignment detection method capable of improving the coating misalignment detection accuracy, an apparatus, a computer device, a computer-readable storage medium, and a computer program product.

In a first aspect, the present application provides a coating misalignment detection method. The method includes:
obtaining a coating type of an electrode plate substrate and determining the number of reference edges according to the coating type;
determining the number of reference edges to be one in the case where the coating type is a one-into-two type;
determining the number of reference edges to be two in the case where the coating type is not a one-into-two type;
determining a corresponding target reference edge of each coating region edge of the electrode plate substrate in the case where the number of reference edges is two;
determining first distance data from coating region edges on a first surface of the electrode plate substrate to a corresponding target reference edge and second distance data from coating region edges on a second surface of the electrode plate substrate to a corresponding target reference edge; and
determining a coating misalignment amount of the electrode plate substrate in a coating process according to the first distance data and the second distance data.

In the above embodiment, when the coating misalignment detection is performed, the number of reference edges that accords with the actual coating conditions is determined according to the coating type of the electrode plate substrate, such that a corresponding target reference edge is subsequently determined for each coating region edge of the electrode plate substrate according to the actual conditions of each coating region edge; obtaining the distance data by detecting the distance from each coating region edge to the corresponding target reference edge thereof that accords with the actual coating conditions and subsequently determining the coating misalignment amount of the electrode plate substrate according to the distance data can reduce the interference from the blank region on the surface of the electrode plate substrate during the coating misalignment detection and effectively improve the accuracy of coating misalignment detection.

In some of the embodiments, determining the corresponding target reference edge of each coating region edge of the electrode plate substrate in the case where the number of reference edges is two includes:
obtaining a plurality of coating region edge pairs of the electrode plate substrate, where each of the coating region edge pairs includes a first coating region edge and a second coating region edge that are respectively positioned on different surfaces of the electrode plate substrate and opposite to each other;
determining edge distances from the first coating region edge and the second coating region edge to two reference edges separately; and
determining a reference edge corresponding to a minimum edge distance as a corresponding target reference edge of the first coating region edge and the second coating region edge in the coating region edge pair.

In the above embodiment, by the dividing of a plurality of coating region edge pairs on the electrode plate substrate and determining the reference edge having the minimum edge distance with the coating region edge in the coating region edge pair as the common target reference edge of the coating region edge pair, the accuracy and stability of the coating misalignment amount calculation can be improved when the coating misalignment amount calculation is performed and the probability of distance detection errors caused by different selected reference edges can be reduced.

In some of the embodiments, determining the reference edge corresponding to the minimum edge distance as the corresponding target reference edge of the first coating region edge and the second coating region edge in the coating region edge pair includes:
ranking the edge distances from the first coating region edge and the second coating region edge to the two reference edges in an ascending order; and
determining a reference edge corresponding to an edge distance ranking first as the corresponding target reference edge of the first coating region edge and the second coating region edge in the coating region edge pair.

In the above embodiment, by ranking the edge distances from the first coating region edge and the second coating region edge to the two reference edges in an ascending order, the minimum edge distance can be quickly determined from the edge distances, and thereby the efficiency and accuracy in determining the target reference edge are effectively improved.

In some of the embodiments, determining the reference edge corresponding to the minimum edge distance as the corresponding target reference edge of the first coating region edge and the second coating region edge in the coating region edge pair includes:
calculating a first average edge distance from the first coating region edge and the second coating region edge to a first reference edge and a second average edge distance from the first coating region edge and the second coating region edge to a second reference edge separately; and
comparing the first average edge distance with the second average edge distance and determining a reference edge corresponding to a minimum average edge distance as the corresponding target reference edge of the first coating region edge and the second coating region edge in the coating region edge pair.

In the above embodiments, by determining the first average edge distance from the first coating region edge and the second coating region edge to the first reference edge and the second average edge distance from the first coating region edge and the second coating region edge to the second reference edge separately, determining the minimum average edge distance according to the first average edge distance and the second average edge distance, and determining the reference edge corresponding to the minimum average edge distance as the corresponding target reference edge, the influence caused by the calculation error of the edge distance can be further eliminated in the process of determining the target reference edge, and the accuracy in determining the target reference edge can be improved.

In some of the embodiments, determining the edge distances from the first coating region edge and the second coating region edge to the two reference edges separately includes:
obtaining first image data resulting from image acquisition aiming at the first surface of the electrode plate substrate and second image data resulting from image acquisition aiming at the second surface of the electrode plate substrate;
determining edge position information of the first coating region edge and the second coating region edge and reference edge position information of the two reference edges according to the first image data and the second image data; and
determining the edge distances from the first coating region edge and the second coating region edge to the two reference edges separately based on the edge position information and the reference edge position information.

In the above embodiment, by obtaining the first image data and the second image data in an image acquisition manner, the controller can quickly determine edge position information representing the edge positions of the coating region edges and reference edge position information representing the positions of the two reference edges according to the first image data and the second image data, and then quickly determine the edge distances from the first coating region edge and the second coating region edge to the two reference edges separately according to the obtained edge position information and reference edge position information, such that the speed and accuracy in determining the edge distances are effectively improved, and thereby the accuracy and efficiency of coating misalignment detection are improved.

In some of the embodiments, obtaining the plurality of coating region edge pairs of the electrode plate substrate includes:
determining a plurality of image combination pairs according to the first image data resulting from image acquisition aiming at the first surface of the electrode plate substrate and the second image data resulting from image acquisition aiming at the second surface of the electrode plate substrate, where each of the image combination pairs includes a first image and a second image with the same acquisition region position;
performing image edge alignment on the first image and the second image; and
matching first coating region edges in the first image to second coating region edges in the second image based on the first image and the second image that are aligned to obtain a plurality of coating region edge pairs corresponding to each image combination pair.

In the above embodiment, by combining the first image and the second image with the same acquisition region position to form an image combination pair and performing image edge alignment on the first image and the second image in each image combination pair, the accuracy of the matching of the first coating region contained in the first image to the second coating region contained in the second image in each image combination pair can be improved, and subsequently, when the coating region edge pairs are determined, the matching of the coating region edges can be directly and quickly performed based on the first image and the second image that are aligned to accurately obtain a plurality of coating region edge pairs corresponding to each image combination pair, such that the speed and accuracy in matching the coating region edges are effectively improved, and thereby the efficiency and accuracy of coating misalignment detection are improved.

In some of the embodiments, determining the plurality of image combination pairs according to the first image data resulting from image acquisition aiming at the first surface of the electrode plate substrate and the second image data resulting from image acquisition aiming at the second surface of the electrode plate substrate includes:
determining an image relative-adjustment parameter for the first image data and the second image data according to an acquisition region interval parameter and an image size parameter for the first image data and the second image data; and
pairing continuous frames of first images contained in the first image data with continuous frames of second images contained in the second image data based on the image relative-adjustment parameter to obtain the plurality of image combination pairs.

In the above embodiment, by determining the image relative-adjustment parameter through the acquisition region interval parameter and the image size parameter and pairing the images in the first image data and the second image data according to the image relative-adjustment parameter, the first image and the second image with the same acquisition region position can be effectively paired, such that the accuracy and efficiency in subsequently determining a plurality of coating region edge pairs based on the paired image combination pairs are improved.

In some of the embodiments, determining the image relative-adjustment parameter for the first image data and the second image data according to the acquisition region interval parameter and the image size parameter for the first image data and the second image data includes:
determining a ratio between the acquisition region interval parameter and the image size parameter for the first image data and the second image data; and
determining the ratio as the image relative-adjustment parameter for the first image data and the second image data.

In the above embodiment, by determining the ratio between the acquisition region interval parameter and the image size parameter for the first image data and the second image data as the image relative-adjustment parameter, the inaccurate image matching caused by different setting positions of the first acquisition device and the second acquisition device can be effectively eliminated, thus improving the matching accuracy of image combination pairs obtained subsequently after image pairing is performed based on the image relative-adjustment parameter.

In some of the embodiments, determining the coating misalignment amount of the electrode plate substrate in the coating process according to the first distance data and the second distance data includes:
determining a distance data set of each image combination pair according to the first distance data and the second distance data, where the distance data set includes a first distance from each first coating region edge in the first image to a corresponding target reference edge and a second distance from each second coating region edge in the second image to a corresponding target reference edge;
determining a single-frame coating misalignment amount of each image combination pair based on first distances and second distances in the distance data set of each image combination pair; and
obtaining an average coating misalignment amount determined according to the single-frame coating misalignment amount of each image combination pair and determining the average coating misalignment amount as the coating misalignment amount of the electrode plate substrate in the coating process.

In the above embodiment, by determining the average coating misalignment amount of the electrode plate substrate according to the single-frame coating misalignment amount of each image combination pair and determining the average coating misalignment amount of the electrode plate substrate as the final coating misalignment amount, the average coating misalignment amount can accurately represent the degree of deviation between the region edges of the first coating region on the first surface of the electrode plate substrate and the region edges of the corresponding second coating region on the second surface of the electrode plate substrate, such that the accuracy and detection efficiency of coating misalignment detection are effectively improved.

In a second aspect, the present application also provides a coating misalignment detection apparatus. The apparatus includes:
a reference edge number determining module configured to obtain a coating type of an electrode plate substrate and determine the number of reference edges according to the coating type, determine the number of reference edges to be one in the case where the coating type is a one-into-two type, and determine the number of reference edges to be two in the case where the coating type is not a one-into-two type;
a target reference edge determining module configured to determine a corresponding target reference edge of each coating region edge of the electrode plate substrate in the case where the number of reference edges is two, where the target reference edge is a reference edge corresponding to a case where the number of blank regions included is minimum when distances from each coating region edge to reference edges are calculated;
a distance data determining module configured to determine first distance data from coating region edges on a first surface of the electrode plate substrate to a corresponding target reference edge and second distance data from coating region edges on a second surface of the electrode plate substrate to a corresponding target reference edge; and
a coating misalignment amount determining module configured to determine a coating misalignment amount of the electrode plate substrate in a coating process according to the first distance data and the second distance data.

In a third aspect, the present application also provides a computer device including a memory and a processor. The memory has a computer program stored therein, and the processor, when executing the computer program, implements the steps of the above method.

In a fourth aspect, the present application also provides a computer-readable storage medium having a computer program stored thereon. The computer program, when executed by a processor, implements the steps of the above method.

In a fifth aspect, the present application also provides a computer program product including a computer program. The computer program, when executed by a processor, implements the steps of the above method.

According to the coating misalignment detection method, the apparatus, the computer device, the storage medium, and the computer program product described above, when the coating misalignment detection is performed, the number of reference edges that accords with the actual coating conditions is first determined based on the coating type of the electrode plate substrate, the number of the reference edges is determined to be one in the case where the coating type is a one-into-two type, and the number of the reference edges is determined to be two in the case where the coating type is not a one-into-two type. In the case where the number of reference edges is determined to be two, a corresponding target reference edge is determined for each coating region edge of the electrode plate substrate according to the actual conditions of each coating region edge. During subsequent distance detection, the coating misalignment amount of the electrode plate substrate is determined based on the first distance data from the coating region edges on the first surface of the electrode plate substrate to the corresponding target reference edge and the second distance data from the coating region edges on the second surface of the electrode plate substrate to the corresponding target reference edge. Compared with the technical solution where a single reference edge is determined at will and the coating misalignment amount of the electrode plate substrate is determined according to this single reference edge, the technical solution of obtaining the distance data by detecting the distance from each coating region edge to the corresponding target reference edge thereof that accords with the actual coating conditions and subsequently determining the coating misalignment amount of the electrode plate substrate according to the distance data can reduce the interference from the blank region on the surface of the electrode plate substrate during the coating misalignment detection and effectively improve the accuracy of coating misalignment detection.

### BRIEF DESCRIPTION OF THE DRAWINGS

To more clearly illustrate the technical solutions in the embodiments of the present application, the drawings required for illustrating the embodiments of the present application are briefly described below. Apparently, the drawings in the following description illustrate merely some embodiments of the present application, and those of ordinary skills in the art may still derive other drawings from these drawings without creative efforts. In the drawings:
FIG. 1 is a schematic structural diagram of a coating deviation correction system according to an embodiment;
FIG. 2 is a schematic flowchart of a coating misalignment detection method according to an embodiment;
FIG. 3 is a schematic flowchart illustrating the step of determining a corresponding target reference edge of each coating region edge of the electrode plate substrate when the number of reference edges is two according to an embodiment;
FIG. 4 is a schematic diagram of the positions of coating region edges and corresponding target reference edges according to an embodiment;
FIG. 5 is a schematic flowchart illustrating the step of determining the edge distances from the first coating region edge and the second coating region edge to two reference edges separately according to an embodiment;
FIG. 6 is a schematic flowchart illustrating the step of obtaining a plurality of coating region edge pairs of the electrode plate substrate according to an embodiment;
FIG. 7 is a schematic diagram of the positions where image acquisition devices are arranged according to an embodiment;
FIG. 8 is a schematic flowchart illustrating the step of determining a plurality of image combination pairs according to first image data resulting from image acquisition aiming at a first surface of the electrode plate substrate and second image data resulting from image acquisition aiming at a second surface of the electrode plate substrate according to an embodiment;
FIG. 9 is a schematic flowchart illustrating the step of determining the coating misalignment amount of the electrode plate substrate in the coating process according to the first distance data and the second distance data according to an embodiment;
FIG. 10 is a schematic diagram illustrating the positioning of the reference edge in the case where the coating type is a one-into-two type according to an embodiment;
FIG. 11 is a schematic diagram illustrating the positioning of the reference edges in the case where the coating type is a one-into-eight type according to an embodiment;
FIG. 12 is a schematic diagram illustrating the positioning of the reference edges in the case where the coating type is a one-into-six type according to an embodiment;
FIG. 13 is a schematic flowchart of a coating misalignment detection method according to another embodiment;
FIG. 14 is a block diagram showing the structure of a coating misalignment detection apparatus according to an embodiment; and
FIG. 15 is a diagram showing the internal structure of a computer device according to an embodiment.

### DETAILED DESCRIPTION

Embodiments of the technical solutions of the present application will be described in detail below with reference to the drawings. The following embodiments are only for illustrating the technical solutions of the present application more clearly, and therefore are only exemplary and do not limit the protection scope of the present application.

Unless otherwise defined, all technical and scientific terms used herein have the same meaning as commonly understood by those skilled in the technical field to which the present application belongs. The terms used herein are only for illustrating the specific embodiments, rather than limiting the present application. The terms "include", "comprise" and "provided with", and any variations thereof in the specification and claims of the present application and the above-mentioned drawing description encompass non-exclusive inclusions.

Reference in the present application to "embodiment" means that a particular feature, structure, or characteristic described in combination with the embodiment can be included in at least one embodiment of the present application. The references of the word in the context of the specification do not necessarily refer to the same embodiment, nor to separate or alternative embodiments exclusive of other embodiments. It will be explicitly and implicitly appreciated by those skilled in the art that the embodiments described herein can be combined with other embodiments.

In the description of the embodiments of the present application, the term "a plurality of" refers to no less than two (including two). Similarly, "a plurality of groups" refers to no less than two groups (including two groups), and "a plurality of pieces" refers to no less than two pieces (including two pieces).

In the description of the embodiments of the present application, the technical terms "center", "longitudinal", "transverse", "length", "width", "thickness", "upper", "lower", "front", "rear", "left", "right", "vertical", "horizontal", "top", "bottom", "inner", "outer", "clockwise" "counterclockwise", "axial", "radial", "circumferential", and the like indicating directional or positional relationships are based on the directional or positional relationships shown in the drawings. They are merely for the convenience of describing the embodiments of the present application and simplifying the description, and are not intended to indicate or imply that the apparatuses or elements referred to must have specific directions or be constructed and operated in specific directions. Therefore, these terms should not be construed as limitations on the embodiments of the present application.

In the description of the embodiments of the present application, unless otherwise clearly specified and defined, the technical terms "install", "interconnect", "connect", "fix", and the like should be interpreted in their broad senses. For example, "connect" may be "fixedly connect", "detachably connect", or "integrally connect"; "mechanically connect" or "electrically connect"; or "directly interconnect", "indirectly interconnect through an intermediate", "communication between interiors of two elements", or "interaction between two elements". For those of ordinary skill in the art, the specific meanings of the above terms in the embodiments of the present application can be interpreted according to the specific condition.

Batteries are widely used as power sources for tools in the fields of energy storage power systems, electric vehicles, and the like. The production of batteries involves many complicated processes such as a stirring process, a coating process, a rolling process, a die-cutting and slitting process, a winding process, an electrode injection process, and a formation process. Among the numerous complex processes, the coating process can be considered to be the most cutting-edge and most important process for new energy batteries.

The coating process is a process of applying one or more layers of liquid to a substrate based on the study of flowing objects, and the stability, uniformity, size, and the like of the coating in the coating process affect the final performance of the battery. The sizes of the coating surfaces A and B, including the position size, the width size, and the misalignment size of the surfaces A and B, have a great influence on the capacity and safety of the battery.

In the coating process, there is the shift of the electrode plate due to mechanical errors, guide roller errors, vibration, fluctuation in the tension of the electrode plate, and the like in the coating device, such that coatings on two surfaces of the electrode plate are misaligned in the coating process. If the misalignment is not found and corrected in time, the performance of the battery may be seriously influenced, and thereby the production cost of the battery is increased. Therefore, the correction of the electrode plate coating is an important step for improving the coating production efficiency. The deviation correction amount in the deviation correcting process needs to be determined according to the misalignment value obtained by the misalignment detection of the coating, and therefore the accuracy of the coating misalignment detection before the deviation correcting directly affects the accuracy and efficiency of the coating deviation correction.

Currently, the technical solution commonly used for coating misalignment detection involves arbitrarily determining a single reference edge and calculating the coating misalignment amount of the electrode plate substrate based on this single reference edge. However, during the determination of the coating misalignment amount, this misalignment detection mode is more or less affected by interference from the blank regions on the surface of the electrode plate substrate, e.g., interference such as wrinkling in the blank regions, which leads to relatively low detection precision of the coating misalignment amount, making it unable to meet the users' subsequent requirements for precise deviation correction.

To enhance the accuracy and efficiency of deviation correction during the coating process of the electrode plate, when the coating misalignment detection is performed, the number of reference edges that accords with the actual coating conditions is first determined based on the coating type of the electrode plate substrate. In the case where the number of reference edges is determined to be two, a corresponding target reference edge is determined for each coating region edge of the electrode plate substrate according to the actual conditions of each coating region edge. The target reference edge can be considered as the reference edge corresponding to the case where the number of blank regions included is minimum when the distances from each coating region edge to the reference edges are measured. During subsequent distance detection, the coating misalignment amount of the electrode plate substrate is determined based on the first distance data from the coating region edges on the first surface of the electrode plate substrate to the corresponding target reference edge and the second distance data from the coating region edges on the second surface of the electrode plate substrate to the corresponding target reference edge. Compared with the technical solution where a single reference edge is determined at will and the coating misalignment amount of the electrode plate substrate is determined according to this single reference edge, the technical solution of obtaining the distance data by detecting the distance from each coating region edge to the corresponding target reference edge thereof that accords with the actual coating conditions and subsequently determining the coating misalignment amount of the electrode plate substrate according to the distance data can reduce the interference from the blank region on the surface of the electrode plate substrate during the coating misalignment detection and effectively improve the accuracy of coating misalignment detection.

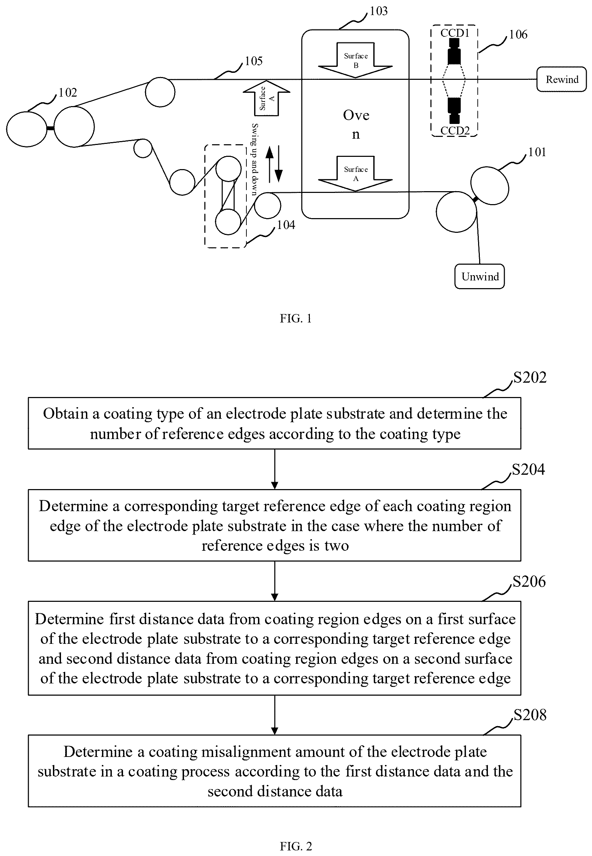

Since the coating misalignment detection is also part of the coating deviation correction, the coating misalignment detection method provided by the embodiments of the present application can be applied to the coating deviation correction system shown in FIG. 1. The coating deviation correction system 100 includes a first die 101, a second die 102, an oven 103, a deviation correction mechanism 104, and a controller (not shown in the figure).

The first die 101 is configured to coat the coating surface A with slurry, the second die 102 is configured to coat the coating surface B with slurry, and the oven 103 is configured to dry the electrode plate substrate 105 coated with slurry. After the coating surfaces A and B are all coated with slurry and dried, the deviation correction detection can be performed to determine whether deviation correction is needed.

The controller may be in communication connection with the first die 101, the second die 102 and the deviation correction mechanism 104, and the data storage system can store the data that needs to be processed by the controller. The data storage system may be integrated on the controller or may be placed on the cloud or other network servers. The controller obtains the coating type of the electrode plate substrate, determines the number of reference edges according to the coating type, determines the corresponding target reference edge of each coating region edge of the electrode plate substrate in the case where the number of the reference edges is two, then determines first distance data from the coating region edges on the first surface of the electrode plate substrate to the corresponding target reference edges and second distance data from the coating region edges on the second surface of the electrode plate substrate to the corresponding target reference edges, and determines the coating misalignment amount of the electrode plate substrate in the coating process according to the first distance data and the second distance data. The controller may be any control chip capable of executing logic processing tasks, such as a microcontroller MCU.

In some embodiments, after the coating surface B is dried by the oven, the manual distance detection may be performed on the surfaces A and B of the electrode plate substrate.

In some other embodiments, an image acquisition device may be further disposed at a position close to the end of the oven 103, and the detection of the edge misalignment value may be performed on the surfaces A and B of the electrode plate substrate in an image acquisition manner. The image acquisition device may be any device capable of implementing image acquisition, such as a charge coupled device camera.

In this embodiment, by disposing a CCD camera detection module 106 at a position close to the end of the oven 103, the image of the coating surface of the electrode plate substrate 105 can be acquired to obtain corresponding image data. Since the misalignment of the corresponding coating regions on the surfaces A and B of the electrode plate substrate 105 needs to be accurately determined during coating deviation correction, the surfaces A and B of the electrode plate substrate 105 need to be simultaneously subjected to image acquisition. Therefore, in this embodiment, the CCD camera detection module 106 further includes a first acquisition device, i.e., CCD1, and a second acquisition device, i.e., CCD2, where the CCD1 is configured to acquire the image of the coating surface A of the electrode plate substrate 105, and the CCD2 is configured to acquire the image of the coating surface B of the electrode plate substrate 105.

In an embodiment, as shown in FIG. 2, a coating misalignment detection method is provided. Taking the application of this method to the controller of the coating deviation correction system in FIG. 1 as an example, the method includes the following steps.

In S202, a coating type of the electrode plate substrate is obtained, and the number of the reference edges is determined according to the coating type.

The electrode plate substrate refers to a lithium ion battery current collector, namely the coating object of the coating process, and the coating process refers to a process of coating one or more layers of slurry on the substrate. The specific material used for the electrode plate substrate can be determined according to actual production requirements. For example, metal semiconductor materials such as copper, aluminum, nickel, and stainless steel can be used, and semiconductor materials such as carbon or composite materials can also be used. It can be understood that the electrode plates of a battery may include a positive electrode plate and a negative electrode plate, and in some embodiments, the electrode plate substrate for producing the positive electrode plate may be an aluminum foil material, and the electrode plate substrate for producing the negative electrode plate may be a copper foil material.

The coating type of the electrode plate substrate refers to the material specification type corresponding to the electrode plate substrate after coating is completed. For example, the coating type of the electrode plate substrate may include one-into-two, one-into-three, one-into-four, one-into-six, one-into-eight, one-into-ten, one-into-twelve, and the like.

The reference edges may be regarded as the edge of the electrode plate substrate along the length direction. One or more reference edges may be arranged for distance detection during misalignment detection, and the number of the reference edges may be determined according to the coating type of the electrode plate substrate.

In some optional embodiments, when the coating misalignment detection is performed, the controller can obtain the coating type of the electrode plate substrate and determine the number of the reference edges according to the coating type.

In some of the embodiments, the controller may determine the number of the reference edges to be two when determining that the electrode plate substrate satisfies the preset multi-reference edge setting condition according to the coating type. The number of the reference edges is determined to be one in the case where it is determined that the electrode plate substrate satisfies the preset single-reference edge setting condition according to the coating type. It can be understood that the multi-reference edge setting condition and the single-reference edge setting condition can be determined by a designer according to the number of the tab regions or the number of the coating regions in the material specification type corresponding to the actual electrode plate substrate.

In S204, in the case where the number of the reference edges is two, the corresponding target reference edge of each coating region edge of the electrode plate substrate is determined.

Since the reference edge may be considered as the edge of the electrode plate substrate along the length direction, when the number of the reference edges is two, it may be considered that the reference edges are respectively provided at both ends of the electrode plate substrate along the length direction.

When the electrode plate substrate is coated, the region coated with the slurry is referred to as a coating region, and the region not coated with the slurry is referred to as a blank region, which may also be referred to as a tab region. Although the blank region is not coated with the slurry, due to the influence of the coating region and the black region by tension and the like during coating, the blank region is likely to wrinkle during the movement. Therefore, to minimize the interference of the wrinkling of the blank region on the detection of the coating misalignment amount, a target reference edge may be selected for the region edges of each coating region according to the actual situation of each coating region. It can be understood that the target reference edge may be considered as the reference edge corresponding to the case where the number of blank regions included is minimum when distances from the coating region edge to the reference edges are calculated subsequently.

In some optional embodiments, the controller determines a corresponding target reference edge for each coating region edge of the electrode plate substrate in the case where the number of the reference edges is determined to be two.

In S206, first distance data from coating region edges on the first surface of the electrode plate substrate to a corresponding target reference edge and second distance data from coating region edges on the second surface of the electrode plate substrate to a corresponding target reference edge are determined.

To improve the coating efficiency, the current commonly used coating process is the double-sided coating process, that is, coating both sides of the electrode plate substrate simultaneously. The first surface and the second surface respectively represent the two corresponding sides of the electrode plate substrate that are coated. For example, when the first surface is the front coating side of the electrode plate substrate in coating, the corresponding second surface can be the back coating side corresponding to the first surface on the electrode plate substrate. Conversely, when the first surface is the back coating side of the electrode plate substrate, the corresponding second surface can be the front coating side corresponding to the first surface.

A coating region of the first surface is referred to as a first coating region, and a coating region of the second surface is referred to as a second coating region. It can be understood that a plurality of first coating regions and a plurality of second coating regions can be arranged on the first surface and the second surface, respectively, and the specific number of the first coating regions and that of the second coating regions are determined according to actual production conditions. In some embodiments, the number of the first coating regions is the same as the number of the second coating regions. The region edge of a coating region may refer to the edge along the length direction of the coating region, which may also be referred to as the longitudinal direction of the electrode plate.

The first distance data includes at least two first distances, and the first distance is a parameter for representing the distance from one region edge of the corresponding first coating region to the target reference edge. Similarly, the second distance data also includes at least two second distances, and the second distance is a parameter for representing the distance from one region edge of the corresponding second coating region to the target reference edge.

In some optional embodiments, after determining the corresponding target reference edge of each coating region edge of the electrode plate substrate, the controller may determine a first distance from each coating region edge on the first surface of the electrode plate substrate to the corresponding target reference edge to obtain first distance data and determine a second distance from each coating region edge on the second surface of the electrode plate substrate to the corresponding target reference edge to obtain second distance data.

In some of the embodiments, the first distance from each coating region edge on the first surface of the electrode plate substrate to the corresponding target reference edge and the second distance from each coating region edge on the second surface of the electrode plate substrate to the corresponding target reference edge can be determined by manual measurements, such as by actually measuring the distance from each coating region edge to the corresponding target reference edge with a length measuring tool.

In some of the embodiments, the first distance from each coating region edge on the first surface of the electrode plate substrate to the corresponding target reference edge and the second distance from each coating region edge on the second surface of the electrode plate substrate to the corresponding target reference edge may be determined by image acquisition of the fist surface and the second surface using an image acquisition device. For example, a charge coupled device (CCD) camera may be used to acquire images of the first surface and the second surface at the same time to obtain image acquisition data, and then the distance detection on the distance from each coating region edge to the corresponding target reference edge is performed based on the image acquisition data to obtain the first distance data and the second distance data.

In S208, a coating misalignment amount of the electrode plate substrate in the coating process is determined according to the first distance data and the second distance data.

The coating misalignment amount can represent the degree of deviation of the region edges of the first coating region on the first surface of the electrode plate substrate and the corresponding second coating region on the second surface of the electrode plate substrate. The coating misalignment amount may be positive or negative, and the positive and negative here do not represent the magnitude of the coating misalignment amount but represent the direction of misalignment, namely the deviation correction direction in subsequent deviation correction treatment. For example, when the coating misalignment amount is determined to be a positive value and the corresponding deviation correction direction is a first direction, the corresponding deviation correction direction should be a second direction when the coating misalignment amount is determined to be a negative value. The first direction is opposite to the second direction, and if a first deviation correction direction is left, a second deviation correction direction is right. If the first deviation correction direction is right, the second deviation correction direction is left.

In some optional embodiments, since the first distance data includes first distances from the coating region edges on the first surface to the corresponding target reference line and the second distance data includes second distances from the coating region edges on the second surface to the corresponding target reference line, the controller may determine a region misalignment value between each first coating region on the first surface and the corresponding second coating region on the second surface according to the first distance data and the second distance data and thereby may determine a coating misalignment amount of the electrode plate substrate in the coating process.

In the above coating misalignment detection method, when the coating misalignment detection is performed, the number of the reference edges that accords with the actual coating conditions of the electrode plate substrate is determined according to the coating type of the electrode plate substrate. In the case where the number of reference edges is determined to be two, a corresponding target reference edge is determined for each coating region edge of the electrode plate substrate according to the actual conditions of each coating region edge. During subsequent distance detection, the coating misalignment amount of the electrode plate substrate is determined based on the first distance data from the coating region edges on the first surface of the electrode plate substrate to the corresponding target reference edge and the second distance data from the coating region edges on the second surface of the electrode plate substrate to the corresponding target reference edge. Compared with the technical solution where a single reference edge is determined at will and the coating misalignment amount of the electrode plate substrate is determined according to this single reference edge, the technical solution of obtaining the distance data by detecting the distance from each coating region edge to the corresponding target reference edge thereof that accords with the actual coating conditions and subsequently determining the coating misalignment amount of the electrode plate substrate according to the distance data can reduce the interference from the blank region on the surface of the electrode plate substrate during the coating misalignment detection and effectively improve the accuracy of coating misalignment detection.

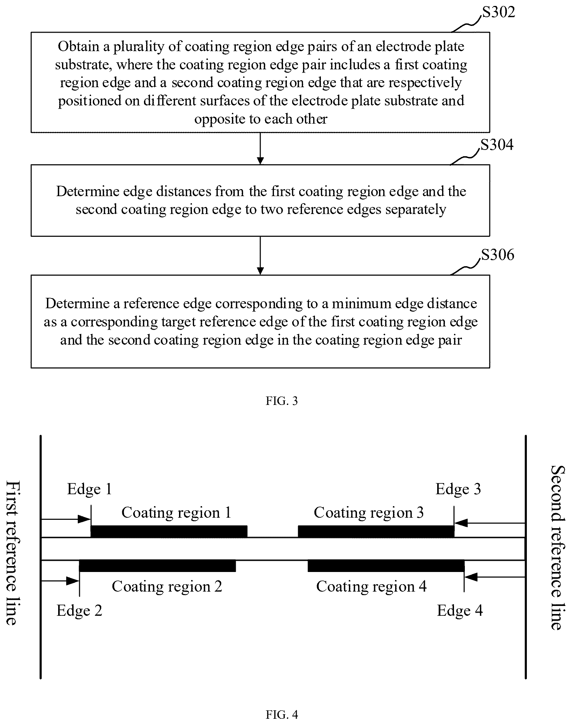

The determination of the target reference edge is a key step for reducing the interference by wrinkling of the blank region. In some embodiments, as shown in FIG. 3, in the case where the number of the reference edges is two, determining the corresponding target reference edge of each coating region edge of the electrode plate substrate includes the following steps.

In S302, a plurality of coating region edge pairs of the electrode plate substrate are obtained, where the coating region edge pair includes a first coating region edge and a second coating region edge that are respectively positioned on different surfaces of the electrode plate substrate and opposite to each other.

Since the coating misalignment detection is mainly a process of detecting the misalignment amount between the coating region on the first surface of the electrode plate substrate and the corresponding coating region on the second surface, the misalignment amount between the coating regions needs to be determined according to the misalignment distance between the corresponding coating region edges of two corresponding coating regions. Therefore, when the target reference edges are determined, it is necessary to set the target reference edges corresponding to the two coating region edges for which the misalignment distance is determined to be the same reference edge. As shown in FIG. 4, for the edge 1 and the edge 2 in the coating region 1 and the coating region 2, the same target reference edge needs to be used for determining the misalignment distance, and for the edge 3 and the edge 4 in the coating region 3 and the coating region 4, another target reference edge needs to be used for determining the misalignment distance.

In some optional embodiments, the controller performs matching treatment on the first coating regions of the first surface of the electrode plate substrate with the second coating regions of the second surface of the electrode plate substrate to determine a corresponding second coating region for each first coating region and thereby obtains coating region pairs, and then matches each first coating region edge to a corresponding second coating region edge according to at least two first coating region edges of the first coating region and at least two second coating region edges of the second coating region of each coating region pair, thus resulting a plurality of coating region edge pairs of the electrode plate substrate.

In S304, edge distances from the first coating region edge and the second coating region edge to the two reference edges are determined separately.

In some optional embodiments, after obtaining the plurality of coating region edge pairs of the electrode plate substrate, the controller may detect the distances from the first coating region edge and the second coating region edge of each coating region edge pair to two reference edges separately to determine the edge distances from the first coating region edge and the second coating region edge to the two reference edges.

In S306, the reference edge corresponding to the minimum edge distance is determined as the corresponding target reference edge of the first coating region edge and the second coating region edge in the coating region edge pair.

In some optional embodiments, after determining the edge distances from the first coating region edge and the second coating region edge to the two reference edges separately, the controller may determine the reference edge corresponding to the minimum edge distance as the common target reference edge of the coating region edge pair, that is, the corresponding target reference edge of the first coating region edge and the second coating region edge in the coating region edge pair.

In some of the embodiments, the controller may rank the obtained edge distances from the first coating region edge and the second coating region edge to the two reference edges in an ascending order and determine the reference edge corresponding to the edge distance ranking first as the common target reference edge of the coating region edge pair.

In the above embodiment, by the dividing of a plurality of coating region edge pairs on the electrode plate substrate and determining the reference edge having the minimum edge distance with the coating region edge in the coating region edge pair as the common target reference edge of the coating region edge pair, the accuracy and stability of the coating misalignment amount calculation can be improved when the coating misalignment amount calculation is performed and the probability of distance detection errors caused by different selected reference edges can be reduced.

In some of the embodiments, after obtaining the edge distances from the first coating region edge and the second coating region edge to the two reference edges, the controller may also calculate a first average edge distance from the first coating region edge and the second coating region edge to the first reference edge and a second average edge distance from the first coating region edge and the second coating region edge to the second reference edge separately, compare the first average edge distance with the second average edge distance, and determine the reference edge corresponding to the minimum average edge distance as the common target reference edge of the coating region edge pair.

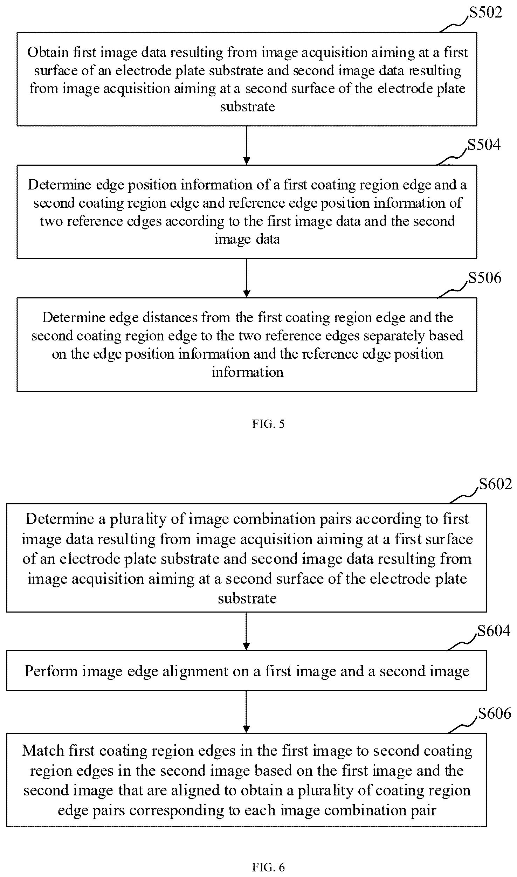

Further, in some embodiments, as shown in FIG. 5, determining the edge distances from the first coating region edge and the second coating region edge to the two reference edges separately includes the following steps.

In S502, first image data resulting from image acquisition aiming at the first surface of the electrode plate substrate and second image data resulting from image acquisition aiming at the second surface of the electrode plate substrate are obtained.

To improve the automation and accuracy of detection, a designer may arrange an image acquisition device in the coating system, and image acquisition of the first surface and the second surface of the electrode plate substrate is performed by using the image acquisition device to obtain the first image data corresponding to the first surface and the second image data corresponding to the second surface.

To improve the detection accuracy, in some embodiments, the first image data and the second image data may be image data obtained by performing a plurality of image acquisitions on the first surface of the electrode plate substrate and the second surface of the electrode plate substrate that are moving by using an image acquisition device in a preset acquisition cycle, where the first image data may include a plurality of frames of continuous first images, and the second image data may include a plurality of frames of continuous second images. It can be understood that the number of image frames specifically contained in the first image data and the second image data is determined according to the sampling cycle preset by the designer and the operating parameters of the image acquisition device.

In some optional embodiments, the controller is in communication connection with the image acquisition device, and the image acquisition device may simultaneously acquire images of the first surface of the electrode plate substrate and the second surface of the electrode plate substrate in response to an image acquisition instruction sent by the controller, thus obtaining the first image data and the second image data.

In S504, edge position information of the first coating region edge and the second coating region edge and reference edge position information of the two reference edges are determined according to the first image data and the second image data.

The edge position information is used for representing the position of each coating region edge of the coating region edge pair in the image, such as the edge mark number and the edge position coordinates. The reference edge position information is used for representing the positions of the two reference edges in the image, such as the reference edge mark number, and the reference edge position coordinates.

In some optional embodiments, after obtaining the first image data and the second image data, the controller may determine the edge position information of the first coating region edge according to a first image in the first image data, determine the edge position information of the second coating region edge according to a second image in the second image data, and determine the reference edge position information of the two reference edges according to the first image and the second image.

In S506, edge distances from the first coating region edge and the second coating region edge to the two reference edges are determined separately based on the edge position information and the reference edge position information.

After obtaining the edge position information and the reference edge position information, the controller may determine the edge distances from the first coating region edge to the two reference edges according to the positions of the two reference edges represented by the reference edge position information and the edge position of the first coating region edge represented by the edge position information, and determine the edge distances from the second coating region edge to the two reference edges according to the positions of the two reference edges represented by the reference edge position information and the edge position of the second coating region edge represented by the edge position information.

In the above embodiment, by obtaining the first image data and the second image data in an image acquisition manner, the controller can quickly determine edge position information representing the edge positions of the coating region edges and reference edge position information representing the positions of the two reference edges according to the first image data and the second image data, and then quickly determine the edge distances from the first coating region edge and the second coating region edge to the two reference edges separately according to the obtained edge position information and reference edge position information, such that the speed and accuracy in determining the edge distances are effectively improved, and thereby the accuracy and efficiency of coating misalignment detection are improved.

In addition to determining the edge distance by image acquisition, the coating region edge pairs may also be determined by image acquisition. In some implementations, based on the coating misalignment detection method shown in FIG. 3 and FIG. 5, as shown in FIG. 6, the S302 of obtaining a plurality of coating region edge pairs of the electrode plate substrate includes the following steps.

In S602, a plurality of image combination pairs are determined according to the first image data resulting from image acquisition aiming at the first surface of the electrode plate substrate and the second image data resulting from image acquisition aiming at the second surface of the electrode plate substrate, where the image combination pair includes a first image and a second image with the same acquisition region position.

It can be understood that to improve the image acquisition quality, the surface of the electrode plate substrate on the roller generally will be subjected to image acquisition to reduce the probability of low acquisition quality caused by the shaking of the electrode plate substrate in the non-roller region in the image acquisition process. However, the first surface and the second surface cannot be present on the roller at the same time in the coating process.

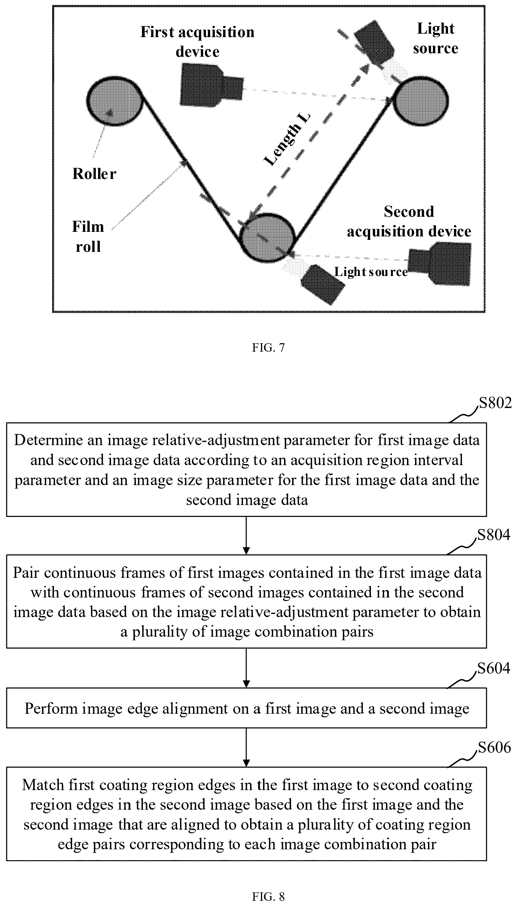

To enable the image acquisition device to simultaneously acquire images of the first surface and the second surface, as shown in FIG. 7, the image acquisition device may include a first acquisition device and a second acquisition device, and the first acquisition device and the second acquisition device respectively and simultaneously acquire images of the surfaces of the electrode plate substrate on the two rollers spaced apart by a certain length L. It can be seen that, due to different acquisition positions, a first coating region corresponding to a first image and a second coating region corresponding to a second image that are acquired by the first acquisition device and the second acquisition device simultaneously are not coating regions opposite to each other on the front and back sides, respectively, of an electrode plate substrate, and the first acquisition device and the second acquisition device are spaced apart by a certain number of pictures.

Since the edge distance is a distance parameter for representing the degree of deviation between a first coating region edge and a corresponding second coating region edge, to improve the accuracy in detecting the edge distance, it is necessary to perform matching and combining on the first images contained in the first image data and the second images contained in the second image data, such that in the image combination pairs resulting from combining, the first coating region contained in a first image and the second coating region contained in a second image are opposite regions, that is, the acquisition region positions of the first image and the second image in the image combination pair are the same.

In some optional embodiments, after obtaining the first image data resulting from image acquisition aiming at the first surface of the electrode plate substrate and the second image data resulting from image acquisition aiming at the second surface of the electrode plate substrate, the controller performs matching and combining on first images and second images in the first image data and the second image data to obtain a plurality of image combination pairs including the first image and the second image with the same acquisition region position.

In S604, image edge alignment is performed on the first image and the second image.

It can be understood that after the controller obtains a plurality of image combination pairs, it is necessary to perform image edge alignment on the first image and the second image in each image combination pair to determine the degree of deviation between the first coating region edges in the first image and the corresponding second coating region edges in the second image more intuitively and accurately.

In S606, first coating region edges in the first image are matched to second coating region edges in the second image based on the first image and the second image that are aligned to obtain a plurality of coating region edge pairs corresponding to each image combination pair.

In some optional embodiments, the controller may match the first coating region edges in the first image to the second coating region edges in the second image based on the first image and the second image that are aligned to obtain a plurality of coating region edge pairs corresponding to each image combination pair.

In the above embodiment, by combining the first image and the second image with the same acquisition region position to form an image combination pair and performing image edge alignment on the first image and the second image in each image combination pair, the accuracy of the matching of the first coating region contained in the first image to the second coating region contained in the second image in each image combination pair can be improved, and subsequently, when the coating region edge pairs are determined, the matching of the coating region edges can be directly and quickly performed based on the first image and the second image that are aligned to accurately obtain a plurality of coating region edge pairs corresponding to each image combination pair, such that the speed and accuracy in matching the coating region edges are effectively improved, and thereby the efficiency and accuracy of coating misalignment detection are improved.

Further, in some embodiments, as shown in FIG. 8, the step S602 of determining the plurality of image combination pairs according to the first image data resulting from image acquisition aiming at the first surface of the electrode plate substrate and the second image data resulting from image acquisition aiming at the second surface of the electrode plate substrate includes the following steps.

In S802, an image relative-adjustment parameter for the first image data and the second image data is determined according to an acquisition region interval parameter and an image size parameter for the first image data and the second image data.

The acquisition region interval parameter refers to an interval parameter between the image acquisition position of the first acquisition device and the image acquisition position of the second acquisition device. For example, it refers to the distance parameter L between a first roller corresponding to the first acquisition device and a second roller corresponding to the second acquisition device.

The image size parameter may be used for representing the size of an acquired image, such as the size of images acquired by the first acquisition device and the second acquisition device. It can be understood that the first acquisition device and the second acquisition device have the same image size parameter.

The image relative-adjustment parameter is an adjustment parameter for image matching of the first images in the first image data and the second images in the second image data and may be the number of image intervals between the first acquisition device and the second acquisition device for acquiring the images. Performing image adjustment pairing on the first image data and the second image data by using the image relative-adjustment parameter can result in a plurality of image combination pairs correspondingly.

In some optional embodiments, the controller determines the image relative-adjustment parameter for the first image data and the second image data according to the acquisition region interval parameter and the image size parameter for the first image data and the second image data.

In some of the embodiments, taking the acquisition region interval parameter being L and the image size parameter being the image length M as an example, the controller may determine F = L/M as the image relative-adjustment parameter for the first image data and the second image data.

S804, continuous frames of first images contained in the first image data are paired with continuous frames of second images contained in the second image data based on the image relative-adjustment parameter to obtain a plurality of image combination pairs.

The image acquisition devices acquire images of the electrode plate substrate in an acquisition cycle, the obtained first image data includes continuous frames of first images, and the obtained second image data includes continuous frames of second images. The specific number of the continuous frames is related to the movement distance of the electrode plate substrate and the image size parameter in the acquisition cycle. Taking the movement distance of the electrode plate substrate being S and the image size parameter being the image length M in the acquisition cycle as an example, in this case, the first image data will include continuous frames of first images with the number of frames N = S/M (N is an integer), and the second image data will also include N continuous frames of second images.

Although the number of frames of the images in the first image data is equal to the number of frames of the images in the second image data, as can be seen from the acquisition environment shown in FIG. 7, when the first acquisition device and the second acquisition device simultaneously acquire the images, the first coating region and the second coating region represented by the acquired first image and second image are definitely not coating regions opposite to each other, that is, the first image and the second image corresponding to the same frame number in the first image data and the second image data represent acquisition of different regions of the electrode plate substrate.

Therefore, after obtaining the image relative-adjustment parameter, the controller may pair the continuous frames of first images contained in the first image data with the continuous frames of second images contained in the second image data based on the image relative-adjustment parameter and match and combine the first image and the second image with the same acquisition region position to obtain an image combination pair, thereby obtaining a plurality of image combination pairs.

For example, when N images are acquired by each of the first acquisition device and the second acquisition device, the first image data includes first images Na1, Na2, Na3, ..., NaN, and the second image data includes second images Nb1, Nb2, Nb3, ..., NbN. If the image relative-adjustment parameter F = 2, it indicates that the number of image intervals between the first acquisition device and the second acquisition device for image acquisition is 2. To allow the regions of the first image and the second image for calculation of the edge misalignment value to be in the same region, Nb1 and Na3 may be determined as an image combination pair, Nb2 and Na4 may be determined as an image combination pair, and so on, that is, the combination pairs that may be obtained from the matching and combining of the first image data and the second image data include (Nb1, Na3), (Nb2, Na4), ..., (Nb(N-2), NaN).

In the above embodiment, by determining the image relative-adjustment parameter through the acquisition region interval parameter and the image size parameter and pairing the images in the first image data and the second image data according to the image relative-adjustment parameter, the first image and the second image with the same acquisition region position can be effectively paired, such that the accuracy and efficiency in subsequently determining a plurality of coating region edge pairs based on the paired image combination pairs are improved.

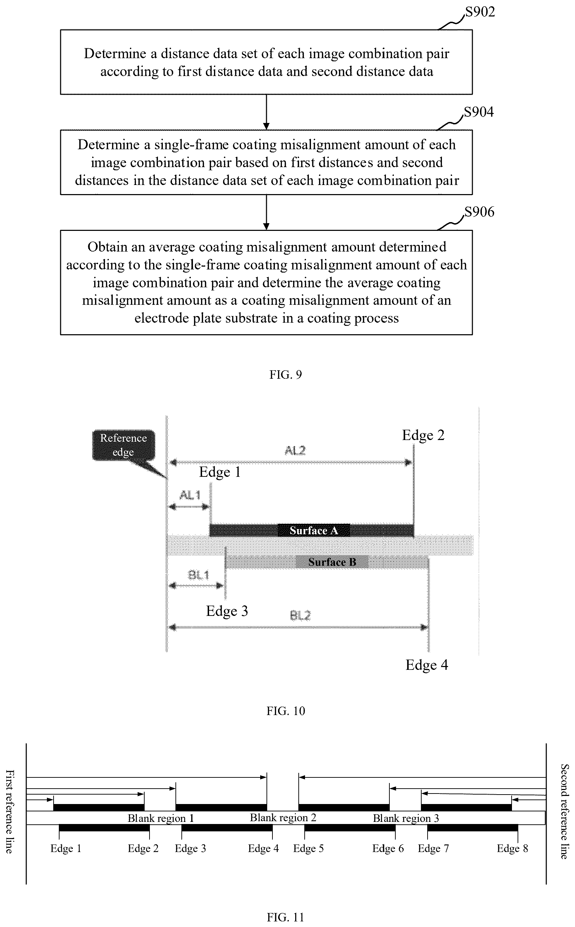

After a plurality of image combination pairs are obtained, the detection speed and detection accuracy of the coating misalignment amount can also be further improved through the image combination pairs. In some embodiments, as shown in FIG. 9, determining the coating misalignment amount of the electrode plate substrate in the coating process according to the first distance data and the second distance data includes the following steps.

In S902, a distance data set of each image combination pair is determined according to the first distance data and the second distance data.

The first distance data is data representing the distances from the coating region edges on the first surface of the electrode plate substrate to the corresponding target reference edge, and in the case where the first image data resulting from image acquisition of the first surface of the electrode plate substrate contains a plurality of frames of continuous first images, the first distance data naturally also contains the first distance from each first coating region edge in each frame of first image to the corresponding target reference edge. Similarly, in the case where the second image data resulting from image acquisition of the second surface of the electrode plate substrate contains a plurality of frames of continuous second images, the second distance data naturally also contains the second distance from each second coating region edge in each frame of second image to the corresponding target reference edge.

Thus, after obtaining the first distance data and the second distance data, the controller may assign a corresponding distance data set to each image combination pair according to the first image and the second image contained in each image combination pair. The distance data set includes the first distance from each first coating region edge in the first image of the image combination pair to the corresponding target reference edge and the second distance from each second coating region edge in the second image to the corresponding target reference edge.

In S904, a single-frame coating misalignment amount of each image combination pair is determined based on first distances and second distances in the distance data set of each image combination pair.

The single-frame coating misalignment amount is used for representing the comprehensive degree of deviation between the coating region on the first surface of the electrode plate substrate and the corresponding coating region on the second surface of the electrode plate substrate in the corresponding first image and second image.

In some optional embodiments, after assigning distance data to each image combination pair to obtain the distance data set, the controller may determine the single-frame coating misalignment amount of each image combination pair based on the first distances and the second distances in the distance data set of each image combination pair.

For example, if in a certain image combination pair, the first distances corresponding to the first image are AL1, AL2, AL3, and AL4, and the second distances corresponding to the second image are BL1, BL2, BL3, and BL4, the initial coating misalignment values of the first surface and the second surface reflected by the first image and the second image are a = AL1 - BL1, b = AL2 - BL2, c = AL3 - BL3, and d = AL4 - BL4, and the controller may determine that the single-frame coating misalignment amount of this image combination pair is: x = (a + b + c + d)/4.

In S906, an average coating misalignment amount determined according to the single-frame coating misalignment amount of each image combination pair is obtained, and the average coating misalignment amount is determined as the coating misalignment amount of the electrode plate substrate in the coating process.

In some optional embodiments, after obtaining the single-frame coating misalignment amount of each image combination pair, the controller may determine an average coating misalignment amount of the electrode plate substrate according to the single-frame coating misalignment amount of each image combination pair and determine the average coating misalignment amount as the coating misalignment amount for representing the degree of deviation between the region edges of the first coating region on the first surface of the electrode plate substrate and the region edges of the corresponding second coating region on the second surface of the electrode plate substrate.

For example, when there are N image combination pairs, the controller may obtain N single-frame coating misalignment amounts including x1, x2, x3, ..., xN, and then the controller may determine the average coating misalignment amount x = (x1 + x2 + x3+ ... + xN)/N as the coating misalignment amount y.

In the above embodiment, by determining the average coating misalignment amount of the electrode plate substrate according to the single-frame coating misalignment amount of each image combination pair and determining the average coating misalignment amount of the electrode plate substrate as the final coating misalignment amount, the average coating misalignment amount can accurately represent the degree of deviation between the region edges of the first coating region on the first surface of the electrode plate substrate and the region edges of the corresponding second coating region on the second surface of the electrode plate substrate, such that the accuracy and detection efficiency of coating misalignment detection are effectively improved.

In the coating misalignment detection method, the determination of the number of the reference edges is a primary step for improving the accuracy of the coating misalignment amount detection. In some embodiments, obtaining a coating type of the electrode plate substrate and determining the number of reference edges according to the coating type includes: determining the number of the reference edges to be one in the case where the coating type is a one-into-two type.

As shown in FIG. 10, in the case where the coating type is the one-into-two type, it can be seen that the number of blank regions included in the calculation process is the same no matter which edge is the reference edge when the edge distance is calculated, and therefore, in the case where the coating type is the one-into-two type, the controller can determine the number of reference edges to be one.

In some other embodiments, obtaining a coating type of the electrode plate substrate and determining the number of reference edges according to the coating type includes: determining the number of the reference edges to be two in the case where the coating type is not a one-into-two type.

It can be understood that in the case where the coating type is one-into-four, one-into-six, one-into-eight, one-into-ten, one-into-twelve, and so on, that is, the coating type is one that is greater than one-into-two, when the edge distance is calculated, the number of blank regions included needs to be considered in the calculation process, and therefore, in the case where the coating type is not a one-into-two type, the controller may determine the number of the reference edges to be two.

In some of the embodiments, the coating type is exemplified as a one-into-eight type. As shown in FIG. 11, in the one-into-eight type, the left two pairs of opposite coating regions include 4 pairs of opposite region edges, namely edges 1, edges 2, edges 3, and edges 4, and the right two pairs of opposite coating regions also include 4 pairs of opposite region edges, namely edges 5, edges 6, edges 7, and edges 8. As is apparent from the drawing, in the one-into-eight type, when the edge distances from the left 4 pairs of region edges to the first reference line are calculated, the number of tab regions included is minimum, and when the edge distances from the right 4 pairs of region edges to the second reference line are calculated, the number of tab regions included is minimum. Therefore, two reference edges need to be provided in the one-into-eight type, and the number of the reference edges is two.

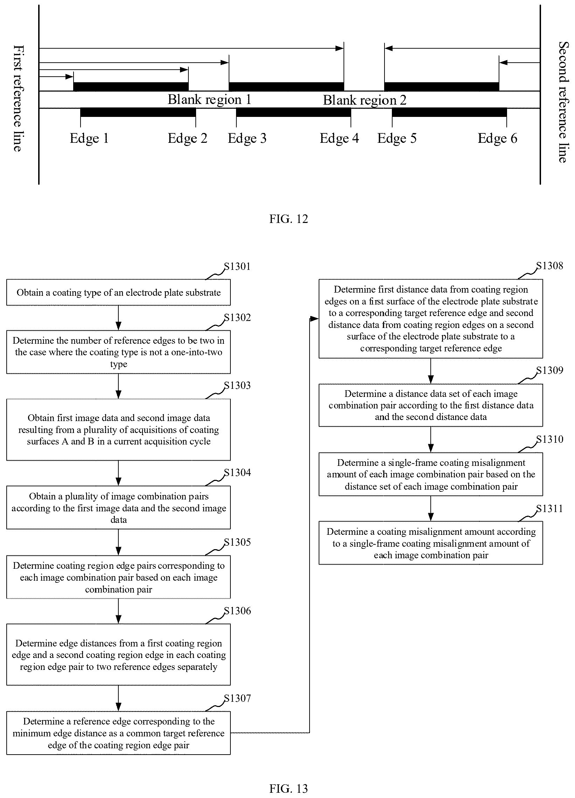

Similarly, in some other embodiments, the coating type is exemplified as a one-into-six type. As shown in FIG. 12, in the one-into-six type, the left pair of opposite coating regions include two pairs of opposite region edges, i.e., edges 1 and edges 2, the middle pair of opposite coating regions include two pairs of opposite region edges, i.e., edges 3 and edges 4, and similarly, the right pair of opposite coating regions include two pairs of opposite region edges, i.e., edges 5 and edges 6. As is apparent from the drawing, in the one-into-six type, when the edge distances from the left two pairs of region edges to the first reference line are calculated, the number of tab regions included is minimum; when the edge distances from the right two pairs of region edges to the second reference line are calculated, the number of tab regions included is minimum; for the middle two pairs of region edges, when the edge distances from the edges 3 to the first reference line are calculated, the number of tab regions included is minimum, and when the edge distances from the edges 4 to the second reference line are calculated, the number of tab regions included is minimum. Therefore, two reference edges also need to be provided in the one-into-eight type, and the number of the reference edges is two.

It should be noted that in the case where the number of the reference edges is two, the deviation correction directions defined by the first reference edge and the second reference edge are opposite. Therefore, if the ± direction is defined based on the misalignment amount calculated according to the first reference edge, a "-" needs to be added to the misalignment amount calculated according to the second reference edge.

In the above embodiment, when the coating misalignment detection is performed, the number of reference edges that accords with the actual coating conditions is determined according to the coating type of the electrode plate substrate, such that a corresponding target reference edge is subsequently determined for each coating region edge of the electrode plate substrate according to the actual conditions of each coating region edge, thus providing a calculation basis for subsequent coating misalignment amount calculation.

In some embodiments, a coating misalignment detection method is provided. Taking the application of this method to the coating deviation correction system in FIG. 1 as an example, as shown in FIG. 13, the coating misalignment detection method specifically includes the following steps.

In S1301, a coating type of an electrode plate substrate is obtained.

It can be understood that the controller can obtain the coating type of the current electrode plate substrate from the server.

In S1302, the number of the reference edges is determined to be two in the case where the coating type is not a one-into-two type.

It can be understood that the controller determines the number of the reference edges to be two in the case where the coating type is not the one-into-two type and determines the number of the reference edges to be one in the case where the coating type is the one-into-two type.

S1303, in the case where the number of reference edges is two, first image data and second image data resulting from a plurality of acquisitions of coating surfaces A and B in a current acquisition cycle are obtained.

After entering an acquisition cycle, the controller generates an image acquisition instruction and sends the image acquisition instruction to the CCD camera detection module. The CCD camera detection module, in response to the image acquisition instruction, controls the CCD1 to acquire images of the coating surface A to obtain first image data and simultaneously controls the CCD2 to acquire images of the coating surface B to obtain second image data.

In S1304, a plurality of image combination pairs are obtained according to the first image data and the second image data.

The controller may generate N groups of pictures according to the first image data and the second image data, and each group of pictures includes a first image and a second image with the same acquisition region position. It can be understood that in the case where the length of each frame of picture is M, the total length of N pictures, L = M × N, is the sampling cycle, where the adopted cycle L is < F, where F is the machine movement distance of the CCD camera to the coating head at surface B, that is, the second die.

In S 1305, coating region edge pairs corresponding to each image combination pair are determined based on each image combination pair.

After obtaining each image combination pair, the controller firstly performs image edge alignment on the first image and the second image in the image combination pair and then matches first coating region edges in the first image to second coating region edges in the second image based on the first image and the second image that are aligned to obtain a plurality of coating region edge pairs corresponding to each image combination pair. It can be understood that the coating region edge pair includes a first coating region edge and a second coating region edge that are respectively positioned on surfaces A and B of the electrode plate substrate and opposite to each other.

In S1306, edge distances from the first coating region edge and the second coating region edge in each coating region edge pair to the two reference edges separately.

In S1307, the reference edge corresponding to the minimum edge distance is determined as a common target reference edge of the coating region edge pair.

After determining the edge distances from the first coating region edge and the second coating region edge in each coating region edge pair to the two reference edges separately, the controller determines the reference edge corresponding to the minimum edge distance as the corresponding target reference edge of the first coating region edge and the second coating region edge in the coating region edge pair.

In S1308, first distance data from the coating region edges on the first surface of the electrode plate substrate to a corresponding target reference edge and second distance data from the coating region edges on the second surface of the electrode plate substrate to a corresponding target reference edge are determined.

In S1309, a distance data set of each image combination pair is determined according to the first distance data and the second distance data.

It can be understood that the controller determines a distance data set of each image combination pair according to the first distance data and the second distance data, and the distance set includes the first distance from each first coating region edge in the first image to the corresponding target reference edge and the second distance from each second coating region edge in the second image to the corresponding target reference edge.

In S1310, a single-frame coating misalignment amount of each image combination pair is determined based on the distance set of each image combination pair.

If in a certain image combination pair, the first distances corresponding to the first image are AL1, AL2, AL3, and AL4, and the second distances corresponding to the second image are BL1, BL2, BL3, and BL4, the initial coating misalignment values of the first surface and the second surface reflected by the first image and the second image are a = AL1 - BL1, b = AL2 - BL2, c = AL3 - BL3, and d = AL4 - BL4, and the controller may determine that the single-frame coating misalignment amount of this image combination pair is: x = (a + b + c + d)/4.

In S1311, a coating misalignment amount is determined according to the single-frame coating misalignment amount of each image combination pair.

After obtaining the single-frame coating misalignment amount of each image combination pair, the controller may determine an average coating misalignment amount of the electrode plate substrate according to the single-frame coating misalignment amount of each image combination pair and determine the average coating misalignment amount as the coating misalignment amount.

It should be understood that although the steps in the flowcharts related to the embodiments described above are displayed sequentially according to the direction of the arrows, these steps are not necessarily executed in the order indicated by the arrows. Unless explicitly stated herein, there is no strict sequential limitation to the execution of these steps, and they can be executed in other orders. Moreover, at least some of the steps in the flowcharts related to the embodiments described above may include multiple steps or stages, which are not necessarily executed at the same time but can be executed at different times. The execution order of these steps or stages is not necessarily sequential; they can be executed in turn or alternately with other steps or at least part of the steps or stages in other steps.

Based on the same inventive concept, embodiments of the present application also provide a coating misalignment detection apparatus for realizing the coating misalignment detection method described above. The implementation solution provided by the apparatus for problem-solving is similar to the one described in the aforementioned method. Therefore, for the specific limitations in one or more embodiments of the coating misalignment detection apparatus provided below, reference can be made to the limitations described above for the coating misalignment detection method, which will not be repeated here.

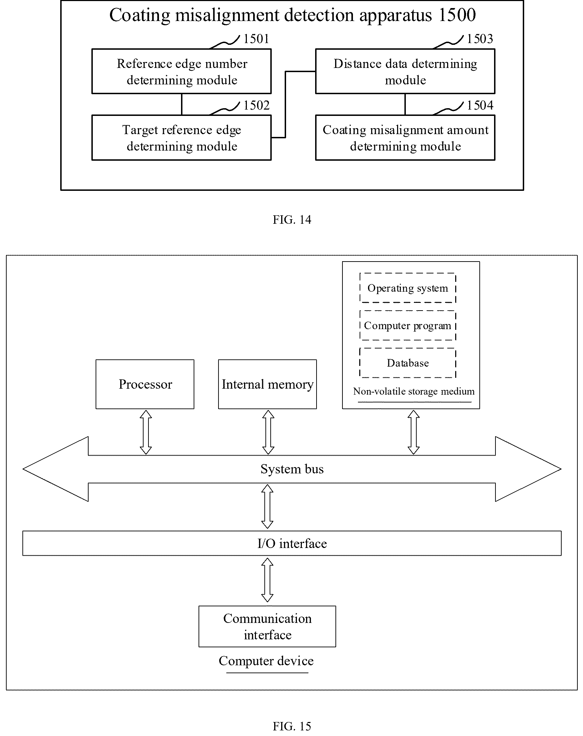

In an embodiment, as shown in FIG. 14, a coating misalignment detection apparatus 1400 is provided. The coating misalignment detection apparatus includes a reference edge number determining module 1401, a target reference edge determining module 1402, a distance data determining module 1403, and a coating misalignment amount determining module 1404.

The reference edge number determining module 1401 is configured to obtain a coating type of an electrode plate substrate and determine the number of reference edges according to the coating type.

The target reference edge determining module 1402 is configured to determine a corresponding target reference edge of each coating region edge of the electrode plate substrate in the case where the number of reference edges is two.

The distance data determining module 1403 is configured to determine first distance data from coating region edges on a first surface of the electrode plate substrate to a corresponding target reference edge and second distance data from coating region edges on a second surface of the electrode plate substrate to a corresponding target reference edge.

The coating misalignment amount determining module 1404 is configured to determine a coating misalignment amount of the electrode plate substrate in the coating process according to the first distance data and the second distance data.

When the coating misalignment detection is performed, the above coating misalignment detection apparatus first determines the number of the reference edges that accords with the actual coating conditions of the electrode plate substrate according to the coating type of the electrode plate substrate. In the case where the number of reference edges is determined to be two, a corresponding target reference edge is determined for each coating region edge of the electrode plate substrate according to the actual conditions of each coating region edge. During subsequent distance detection, the coating misalignment amount of the electrode plate substrate is determined based on the first distance data from the coating region edges on the first surface of the electrode plate substrate to the corresponding target reference edge and the second distance data from the coating region edges on the second surface of the electrode plate substrate to the corresponding target reference edge. Compared with determining a single reference edge at will and determining the coating misalignment amount of the electrode plate substrate according to this single reference edge, obtaining the distance data by detecting the distance from each coating region edge to the corresponding target reference edge thereof that accords with the actual coating conditions and subsequently determining the coating misalignment amount of the electrode plate substrate according to the distance data can reduce the interference from the blank region on the surface of the electrode plate substrate during the coating misalignment detection and effectively improve the accuracy of coating misalignment detection.

In some embodiments, the target reference edge determining module is further configured to: obtain a plurality of coating region edge pairs of the electrode plate substrate, where the coating region edge pair includes a first coating region edge and a second coating region edge that are respectively positioned on different surfaces of the electrode plate substrate and opposite to each other; determine edge distances from the first coating region edge and the second coating region edge to two reference edges separately; and determine a reference edge corresponding to a minimum edge distance as a corresponding target reference edge of the first coating region edge and the second coating region edge in the coating region edge pair.

In some embodiments, the target reference edge determining module is further configured to: rank the edge distances from the first coating region edge and the second coating region edge to the two reference edges in an ascending order, and determine a reference edge corresponding to an edge distance ranking first as the corresponding target reference edge of the first coating region edge and the second coating region edge in the coating region edge pair.

In some embodiments, the target reference edge determining module is further configured to: calculate a first average edge distance from the first coating region edge and the second coating region edge to a first reference edge and a second average edge distance from the first coating region edge and the second coating region edge to a second reference edge separately; and compare the first average edge distance with the second average edge distance and determine a reference edge corresponding to a minimum average edge distance as the corresponding target reference edge of the first coating region edge and the second coating region edge in the coating region edge pair.

In some embodiments, the target reference edge determining module is further configured to: obtain first image data resulting from image acquisition aiming at the first surface of the electrode plate substrate and second image data resulting from image acquisition aiming at the second surface of the electrode plate substrate; determine edge position information of the first coating region edge and the second coating region edge and reference edge position information of the two reference edges according to the first image data and the second image data; and determine the edge distances from the first coating region edge and the second coating region edge to the two reference edges separately based on the edge position information and the reference edge position information.

In some embodiments, the target reference edge determining module is further configured to: determine a plurality of image combination pairs according to the first image data resulting from image acquisition aiming at the first surface of the electrode plate substrate and the second image data resulting from image acquisition aiming at the second surface of the electrode plate substrate, where the image combination pair includes a first image and a second image with the same acquisition region position; perform image edge alignment on the first image and the second image; and match first coating region edges in the first image to second coating region edges in the second image based on the first image and the second image that are aligned to obtain a plurality of coating region edge pairs corresponding to each image combination pair.

In some embodiments, the target reference edge determining module is further configured to: determine an image relative-adjustment parameter for the first image data and the second image data according to an acquisition region interval parameter and an image size parameter for the first image data and the second image data; and pair continuous frames of first images contained in the first image data with continuous frames of second images contained in the second image data based on the image relative-adjustment parameter to obtain the plurality of image combination pairs.

In some embodiments, the target reference edge determining module is further configured to: determine a ratio between the acquisition region interval parameter and the image size parameter for the first image data and the second image data; and determine the ratio as the image relative-adjustment parameter for the first image data and the second image data.

In some embodiments, the coating misalignment amount determining module is further configured to: determine a distance data set of each image combination pair according to the first distance data and the second distance data, where the distance data set includes a first distance from each first coating region edge in the first image to a corresponding target reference edge and a second distance from each second coating region edge in the second image to a corresponding target reference edge; determine a single-frame coating misalignment amount of each image combination pair based on first distances and second distances in the distance data set of each image combination pair; and obtain an average coating misalignment amount determined according to the single-frame coating misalignment amount of each image combination pair and determine the average coating misalignment amount as the coating misalignment amount of the electrode plate substrate in the coating process.

In some embodiments, the reference edge number determining module is further configured to determine the number of reference edges as one in the case where the coating type is a one-into-two type and determine the number of the reference edges to be two in the case where the coating type is not a one-into-two type.

The modules in the above coating misalignment detection apparatus may be entirely or partially implemented by software, hardware, and a combination thereof. The above modules may be embedded as hardware into or independent of a processor in the computer device or may also be stored as software in a memory in the computer device to enable the processor to call and execute operations corresponding to the modules.

In an embodiment, provided is a computer device. The computer device may be a controller, and the internal structure thereof may be as shown in FIG. 15. The computer device includes a processor, a memory, and a network interface connected by a system bus. The processor of the computer device is configured to provide computing and control capabilities. The memory of the computer device includes a non-volatile storage medium and an internal memory. The non-volatile storage medium stores an operating system, a computer program, and a database. The internal memory provides an environment for the operation of the operating system and the computer program in the non-volatile storage medium. The database of the computer device is used for storing data such as the coating type, the number of reference edges, the first distance data, the second distance data, and the coating misalignment amount. The network interface of the computer device is used for communicating with an external terminal through a network connection. The computer program, when executed by the processor, implements a coating misalignment detection method.

It can be understood by those skilled in the art that the structure shown in FIG. 15 is a block diagram of only part of the structure associated with the embodiments of the present application and is not intended to limit the computer devices to which the embodiments of the present application are applied, and that a specific computer device may include more or less components than those shown, or may have certain components combined, or may have a different arrangement of components.

In an embodiment, provided is a computer device including a memory and a processor. The memory has a computer program stored therein, and the processor, when executing the computer program, implements specific implementation steps of the above coating misalignment detection method.

In an embodiment, provided is a computer-readable storage medium having a computer program stored thereon. The computer program, when executed by a processor, implements specific implementation steps of the above coating misalignment detection method.

In an embodiment, provided is a computer program product including a computer program. The computer program, when executed by a processor, implements specific implementation steps of the above coating misalignment detection method.

It should be noted that the user information (including but not limited to user device information, user personal information, etc.) and data (including but not limited to data for analysis, stored data, displayed data, etc.) involved in the present application are information and data authorized by the users or sufficiently authorized by each party.

It can be understood by those of ordinary skill in the art that all or part of the processes in the methods of the above embodiments may be implemented by executing a computer program to instruct the relevant hardware. The computer program may be stored in a non-volatile computer-readable storage medium. The computer program, when executed, may include the processes in the methods of the above embodiments. Any reference to a memory, a database, or other media used in the embodiments provided in the present application can include at least one of a non-volatile memory and a volatile memory. The non-volatile memory may include a read-only memory (ROM), a magnetic tape, a floppy disk, a flash memory, an optical storage, a high-density embedded non-volatile memory, a resistive random access memory (ReRAM), a magnetoresistive random access memory (MRAM), a ferroelectric random access memory (FRAM), a phase change memory (PCM), a graphene memory, and the like. The volatile memory may include a random access memory (RAM), an external cache memory, and the like. By way of illustration and not limitation, RAM may be in various forms, such as a static random access memory (SRAM) or a dynamic random access memory (DRAM). The database involved in the embodiments provided in the present application may include at least one of a relational database and a non-relational database. The non-relational database may include, but is not limited to, a blockchain-based distributed database. The processor involved in the embodiments provided in the present application may be, but is not limited to, a general-purpose processor, a central processing unit, a graphics processor, a digital signal processor, a programmable logic unit, a data processing logic unit based on quantum computation, etc.

The technical features of the above embodiments may be arbitrarily combined. For the sake of concise description, not all possible combinations of the technical features in the above embodiments have been described. However, as long as there is no contradiction between the combinations of these technical features, they should be considered to be within the scope of the specification.

The above embodiments represent only several implementations of the present application, and the description thereof is specific and detailed, but this should not be understood as limiting the patent scope of the present application. It should be noted that several variations and improvements may be made by those of ordinary skill in the art without departing from the concept of the present application, and these variations and improvements are all within the scope of protection of the present application. Therefore, the scope of protection of the present application should be subject to the appended claims.

## Claims

1. A coating misalignment detection method, the method comprising:
obtaining a coating type of an electrode plate substrate and determining a number of reference edges according to the coating type;
determining the number of reference edges to be one in a case where the coating type is a one-into-two type;
determining the number of reference edges to be two in a case where the coating type is not a one-into-two type;
determining a corresponding target reference edge of each coating region edge of the electrode plate substrate in a case where the number of reference edges is two, wherein the target reference edge is a reference edge corresponding to a case where a number of blank regions comprised is minimum when distances from each coating region edge to reference edges are calculated;
determining first distance data from coating region edges on a first surface of the electrode plate substrate to a corresponding target reference edge and second distance data from coating region edges on a second surface of the electrode plate substrate to a corresponding target reference edge; and
determining a coating misalignment amount of the electrode plate substrate in a coating process according to the first distance data and the second distance data.

2. The method according to claim 1, wherein determining the corresponding target reference edge of each coating region edge of the electrode plate substrate in the case where the number of reference edges is two comprises:
obtaining a plurality of coating region edge pairs of the electrode plate substrate, wherein each of the coating region edge pairs comprises a first coating region edge and a second coating region edge that are respectively positioned on different surfaces of the electrode plate substrate and opposite to each other;
determining edge distances from the first coating region edge and the second coating region edge to two reference edges separately; and
determining a reference edge corresponding to a minimum edge distance as a corresponding target reference edge of the first coating region edge and the second coating region edge in the coating region edge pair.

3. The method according to claim 2, wherein determining the reference edge corresponding to the minimum edge distance as the corresponding target reference edge of the first coating region edge and the second coating region edge in the coating region edge pair comprises:
ranking the edge distances from the first coating region edge and the second coating region edge to the two reference edges in an ascending order; and
determining a reference edge corresponding to an edge distance ranking first as the corresponding target reference edge of the first coating region edge and the second coating region edge in the coating region edge pair.

4. The method according to claim 2, wherein determining the reference edge corresponding to the minimum edge distance as the corresponding target reference edge of the first coating region edge and the second coating region edge in the coating region edge pair comprises:
calculating a first average edge distance from the first coating region edge and the second coating region edge to a first reference edge and a second average edge distance from the first coating region edge and the second coating region edge to a second reference edge separately; and
comparing the first average edge distance with the second average edge distance and determining a reference edge corresponding to a minimum average edge distance as the corresponding target reference edge of the first coating region edge and the second coating region edge in the coating region edge pair.

5. The method according to any one of claims 2 to 4, wherein determining the edge distances from the first coating region edge and the second coating region edge to the two reference edges separately comprises:
obtaining first image data resulting from image acquisition aiming at the first surface of the electrode plate substrate and second image data resulting from image acquisition aiming at the second surface of the electrode plate substrate;
determining edge position information of the first coating region edge and the second coating region edge and reference edge position information of the two reference edges according to the first image data and the second image data; and
determining the edge distances from the first coating region edge and the second coating region edge to the two reference edges separately based on the edge position information and the reference edge position information.

6. The method according to any one of claims 2 to 5, wherein obtaining the plurality of coating region edge pairs of the electrode plate substrate comprises:
determining a plurality of image combination pairs according to the first image data resulting from image acquisition aiming at the first surface of the electrode plate substrate and the second image data resulting from image acquisition aiming at the second surface of the electrode plate substrate, wherein each of the image combination pairs comprises a first image and a second image with a same acquisition region position;
performing image edge alignment on the first image and the second image; and
matching first coating region edges in the first image to second coating region edges in the second image based on the first image and the second image that are aligned to obtain a plurality of coating region edge pairs corresponding to each image combination pair.

7. The method according to claim 6, wherein determining the plurality of image combination pairs according to the first image data resulting from image acquisition aiming at the first surface of the electrode plate substrate and the second image data resulting from image acquisition aiming at the second surface of the electrode plate substrate comprises:
determining an image relative-adjustment parameter for the first image data and the second image data according to an acquisition region interval parameter and an image size parameter for the first image data and the second image data; and
pairing continuous frames of first images contained in the first image data with continuous frames of second images contained in the second image data based on the image relative-adjustment parameter to obtain the plurality of image combination pairs.

8. The method according to claim 7, wherein determining the image relative-adjustment parameter for the first image data and the second image data according to the acquisition region interval parameter and the image size parameter for the first image data and the second image data comprises:
determining a ratio between the acquisition region interval parameter and the image size parameter for the first image data and the second image data; and
determining the ratio as the image relative-adjustment parameter for the first image data and the second image data.

9. The method according to any one of claims 6 to 8, wherein determining the coating misalignment amount of the electrode plate substrate in the coating process according to the first distance data and the second distance data comprises:
determining a distance data set of each image combination pair according to the first distance data and the second distance data, wherein the distance data set comprises a first distance from each first coating region edge in the first image to a corresponding target reference edge and a second distance from each second coating region edge in the second image to a corresponding target reference edge;
determining a single-frame coating misalignment amount of each image combination pair based on first distances and second distances in the distance data set of each image combination pair; and
obtaining an average coating misalignment amount determined according to the single-frame coating misalignment amount of each image combination pair and determining the average coating misalignment amount as the coating misalignment amount of the electrode plate substrate in the coating process.

10. A coating misalignment detection apparatus, the apparatus comprising:
a reference edge number determining module configured to obtain a coating type of an electrode plate substrate and determine a number of reference edges according to the coating type, determine the number of reference edges to be one in a case where the coating type is a one-into-two type, and determine the number of reference edges to be two in a case where the coating type is not a one-into-two type;
a target reference edge determining module configured to determine a corresponding target reference edge of each coating region edge of the electrode plate substrate in a case where the number of reference edges is two, wherein the target reference edge is a reference edge corresponding to a case where a number of blank regions comprised is minimum when distances from each coating region edge to reference edges are calculated;
a distance data determining module configured to determine first distance data from coating region edges on a first surface of the electrode plate substrate to a corresponding target reference edge and second distance data from coating region edges on a second surface of the electrode plate substrate to a corresponding target reference edge; and
a coating misalignment amount determining module configured to determine a coating misalignment amount of the electrode plate substrate in a coating process according to the first distance data and the second distance data.

11. The apparatus according to claim 10, wherein the target reference edge determining module is further configured to:
obtain a plurality of coating region edge pairs of the electrode plate substrate, wherein each of the coating region edge pairs comprises a first coating region edge and a second coating region edge that are respectively positioned on different surfaces of the electrode plate substrate and opposite to each other;
determine edge distances from the first coating region edge and the second coating region edge to two reference edges separately; and
determine a reference edge corresponding to a minimum edge distance as a corresponding target reference edge of the first coating region edge and the second coating region edge in the coating region edge pair.

12. The apparatus according to claim 11, wherein the target reference edge determining module is further configured to:
rank the edge distances from the first coating region edge and the second coating region edge to the two reference edges in an ascending order; and
determine a reference edge corresponding to an edge distance ranking first as the corresponding target reference edge of the first coating region edge and the second coating region edge in the coating region edge pair.

13. The apparatus according to claim 11, wherein the target reference edge determining module is further configured to:
calculate a first average edge distance from the first coating region edge and the second coating region edge to a first reference edge and a second average edge distance from the first coating region edge and the second coating region edge to a second reference edge separately; and
compare the first average edge distance with the second average edge distance and determine a reference edge corresponding to a minimum average edge distance as the corresponding target reference edge of the first coating region edge and the second coating region edge in the coating region edge pair.

14. The apparatus according to any one of claims 11 to 13, wherein the target reference edge determining module is further configured to:
obtain first image data resulting from image acquisition aiming at the first surface of the electrode plate substrate and second image data resulting from image acquisition aiming at the second surface of the electrode plate substrate;
determine edge position information of the first coating region edge and the second coating region edge and reference edge position information of the two reference edges according to the first image data and the second image data; and
determine the edge distances from the first coating region edge and the second coating region edge to the two reference edges separately based on the edge position information and the reference edge position information.

15. The apparatus according to any one of claims 11 to 14, wherein the target reference edge determining module is further configured to:
determine a plurality of image combination pairs according to the first image data resulting from image acquisition aiming at the first surface of the electrode plate substrate and the second image data resulting from image acquisition aiming at the second surface of the electrode plate substrate, wherein each of the image combination pairs comprises a first image and a second image with a same acquisition region position;
perform image edge alignment on the first image and the second image; and
match first coating region edges in the first image to second coating region edges in the second image based on the first image and the second image that are aligned to obtain a plurality of coating region edge pairs corresponding to each image combination pair.

16. The apparatus according to claim 15, wherein the target reference edge determining module is further configured to:
determine an image relative-adjustment parameter for the first image data and the second image data according to an acquisition region interval parameter and an image size parameter for the first image data and the second image data; and
pair continuous frames of first images contained in the first image data with continuous frames of second images contained in the second image data based on the image relative-adjustment parameter to obtain the plurality of image combination pairs.

17. The apparatus according to claim 16, wherein the target reference edge determining module is further configured to:
determine a ratio between the acquisition region interval parameter and the image size parameter for the first image data and the second image data; and
determine the ratio as the image relative-adjustment parameter for the first image data and the second image data.

18. The apparatus according to any one of claims 15 to 17, wherein the coating misalignment amount determining module is further configured to:
determine a distance data set of each image combination pair according to the first distance data and the second distance data, wherein the distance data set comprises a first distance from each first coating region edge in the first image to a corresponding target reference edge and a second distance from each second coating region edge in the second image to a corresponding target reference edge;
determine a single-frame coating misalignment amount of each image combination pair based on first distances and second distances in the distance data set of each image combination pair; and
obtain an average coating misalignment amount determined according to the single-frame coating misalignment amount of each image combination pair and determine the average coating misalignment amount as the coating misalignment amount of the electrode plate substrate in the coating process.

19. A computer device comprising a memory and a processor, the memory having a computer program stored therein, wherein the processor, when executing the computer program, implements the steps of the method according to any one of claims 1 to 9.

20. A computer-readable storage medium having a computer program stored thereon, wherein the computer program, when executed by a processor, implements the steps of the method according to any one of claims 1 to 9.

21. A computer program product comprising a computer program, wherein the computer program, when executed by a processor, implements the steps of the method according to any one of claims 1 to 9.
